# NEUE EUROPÄISCHE PATENTSCHRIFT

(11) **EP 1 610 685 B2**
(45) Veröffentlichungstag und Bekanntmachung des Hinweises auf die Entscheidung über den Einspruch: **29.04.2015**
(45) Hinweis auf die Patenterteilung: 08.02.2012
(21) Anmeldenummer: 04722801.0
(22) Anmeldetag: 24.03.2004
(51) Int. Cl.: A61B 6/03, G03B 42/04, A61B 6/14

(54) **Röntgeneinrichtung und röntgenstrahlenempfindliche Kamera für Panoramaschichtaufnahmen und 3d-Aufnahmen**
X-ray device and X-ray sensitive camera for taking panoramic tomographic images and 3D images
Dispositif radiographique et caméra sensible aux rayons X pour prises de vues tomographiques panoramiques et prises de vues en 3D

(30) Priorität: 24.03.2003 DE 10313110
(43) Veröffentlichungstag der Anmeldung: 04.01.2006
(73) Patentinhaber: Sirona Dental Systems GmbH, 64625 Bensheim (DE)
(72) Erfinder: ERHARDT, Norbert, 67549 Worms (DE); GÜNTHER, Werner, 64625 Bensheim (DE)
(74) Vertreter: Sommer, Peter
(86) Internationale Anmeldenummer: PCT/DE2004/000620
(87) Internationale Veröffentlichungsnummer: WO 2004/084728

(56) Entgegenhaltungen:
- EP-A- 0 229 971
- EP-A- 1 219 244
- EP-A2- 0 858 773
- DE-A1- 19 941 668
- JP-A- H 119 579
- JP-A- H09 135 829
- US-A- 6 118 842
- US-A1- 2003 030 721
- US-B1- 6 379 041
- US-B1- 6 466 641
- J. HAMASAKI AND K. YOKOTA: "Direct recording and reconstruction of 3D X-ray images" APPLIED OPTICS, Bd. 17, Nr. 19, 1. Oktober 1978 (1978-10-01), Seiten 3125-3132, XP001182639
- SSU-KUANG CHEN, CHUNG-MING CHEN: "A hight resolution CT system using intraoral x-ray detector" CAR'98, 1998, Seiten 798-802, XP009035091

## Beschreibung

Die Erfindung betrifft eine Röntgeneinrichtung mit einer für Röntgenstrahlen empfindlichen Kamera, welche einen für Röntgenstrahlen empfindlichen Bildempfänger zur Erstellung einer Schichtaufnahme umfasst.

Ein derartige Röntgeneinrichtung wird zur Erstellung von dentalen Panoramaschichtaufnahmen verwendet.

### Stand der Technik

Aus der EP 0 229 971 ist ein zahnärztliches Röntgendiagnostikgerät zur Erstellung von Panorama-Schichtaufnahmen vom Kiefer eines Patienten bekannt. Zusätzlich zu den Panoramaschichtaufnahmen (PAN-Aufnahmen) können auf dem Film von einem oder mehreren beliebig wählbaren Abschnitten des Kiefers Abbildungen in mehreren Schichtlagen nebeneinander gemacht werden (Multischicht-Aufnahmen). Darüber hinaus ist ein Filmkassettenhalter so an einer den Röntgenstrahler tragenden Dreheinheit befestigt, dass er aus einer Gebrauchsstellung in eine Nichtgebrauchslage verschwenkt werden kann, mit welcher das Erstellen von Fernröntgenaufnahmen (Ceph-Aufnahmen) ermöglicht wird, da die Röntgenstrahlenquelle ungestört an dem Filmkassettenhalter vorbei strahlt.

Aus der EP 0 632 994 A1 ist eine Röntgendiagnostikeinrichtung zur Erstellung von Röntgenaufnahmen von Körperteile eines Patienten bekannt, bei der eine Zeilendetektor-Kamera mit einem Röntgenstrahlen-Detektor vorgesehen ist, dessen Breite der Breite bzw. der Länge des aufzunehmenden Körperteils angepasst ist. Die Zeilendetektor-Kamera kann über Verstellmittel zusammen mit der Strahlenquelle entlang des aufzunehmenden Körperteils bewegt werden. Die Röntgendiagnostikeinrichtung kann dabei sowohl zur Erstellung einer PAN-Aufnahme als auch zur Erstellung einer Fernaufnahme (Ceph) ausgebildet sein, wobei die Zeilendetektor-Kamera zur Erstellung der jeweiligen Aufnahme umsteckbar ist und hierzu ein Anschlussteil aufweist, welches Anschlussmittel für eine lösbare mechanische und elektrische Verbindung mit einem Halter beinhalten. Darüber hinaus sind verschieden Möglichkeiten zur Führung des Röntgenstrahlfächers bei der Erstellung der Fernaufnahme mit einem bewegten Strahler oder einer Primärblende oder beiden zusammen offenbart.

Eine umsteckbare Kamera ist im Detail in der EP 0 634 671 A1 beschrieben, wobei insbesondere auf die lösbare Befestigung der Kamera an einem Halter abgestellt wird.

Aus der EP 0 858 773 A2 ist eine Detektoranordnung zur Erstellung von Röntgenaufnahmen bekannt, welche aus Detektoren in den Abmessungen des Detektors eines Intraoralsensors besteht. Die Detektoranordnung ist so ausgebildet, dass Transversal-Schichtaufnahmen (TSA-Aufnahmen) erstellt werden können, wobei die Detektoranordnung innerhalb der Zeilendetektorkamera in Richtung Ihrer Längsachse verstellbar gehaltert ist. Die Detektorelemente können mittels einer Verstelleinrichtung entlang der Detektorhauptachse verstellt werden.

Die in der EP 0 858 773 A2 verwendeten Sensoren zur Erstellung einer PAN- oder Ceph-Aufnahme weisen typischerweise 135 bis 180 mm in der Bildhöhe und ca. 6 mm in der Bildbreite auf. Die für die Erstellung von TSA-Aufnahmen verwendeten Sensoren haben typischerweise Abmessungen von etwa 30 x 20 mm. Der Breitenunterschied resultiert daraus, dass bei der Panorama-Schichtaufnahme eine Schichtdicke (Tiefenschärfebereich) der scharfen Schicht mindestens so groß wie die Dicke des aufgenommenen Objektes gewünscht ist, wohingegen bei der TSA-Aufnahme die Schichtdicke (Tiefenschärfebereich) der scharfen Schicht etwa 1 bis 3 mm beträgt.

Die aus der EP 0 858 773 A2 hervorgegangene EP 1 219 244 B1 offenbart ein Verfahren und eine Einrichtung zur Herstellung von Röntgenaufnahmen von Körperteilen eines Menschen. Dazu ist eine Detektoranordnung mit mindestens einem Detektor vorgesehen. Eine Bildaufnahme erfolgt in mehreren, zeitlich getrennten Abschnitten. Die Detektoreinrichtung wird zwischen den Röntgenaufnahmen entlang der Längsachse der Detektorfläche verstellt, um einen größeren Aufnahmebereich mit vergleichsweise kleinen Sensoren zu erzielen. Verschiedene Aufnahmemodi sind möglich.

Die Detektoranordnung kann aus mehreren Detektoren aufgebaut sein, wobei diese Detektoren in vertikaler Richtung übereinander angeordnet sind. Diese Detektoren können mittels einer Verstelleinrichtung in der vertikalen Richtung verstellt werden, um einen einzelnen Zeilensensor zu simulieren.

Aus den aufgenommenen Röntgenaufnahmen kann eine transversale Schichtaufnahme (TSA) erstellt werden. Zur Bilderfassung wird ein TDI-Verfahren verwendet. Alternativ dazu werden die Aufnahmen nach einer Zwischenspeicherung von einer Signalverarbeitungssoftware zu einer transversalen Schichtaufnahme zusammengesetzt. Die vertikale Verstellrichtung der Sensoren ist dabei senkrecht zur TDI-Richtung, so dass die Sensoren nur in einem einzigen Aufnahmemodus für Schichtaufnahmen verwendet werden.

Die US 2003 0030721 A1 offenbart eine Digitalkamera für eine herkömmliche Röntgeneinrichtung, die Panoramaaufnahmen und cephalometrischen Aufnahmen ermöglicht, wobei in der Kamera mehrere Sensoren verbaut sind, die sich in einer Richtung gegenseitig überlappen. Ein Sensor wird dabei für tomographische Aufnahmen benutzt und mindestens ein weiterer Sensor für Durchleuchtungsaufnahmen.

Der Verstellung zwischen einem Modus für tomografische Aufnahmen und einem Modus für Durchleuchtungsaufnahmen erfolgt mittels eines Kollimators. Der Kollimator weist dabei eine größere Öffnung für cephalometrische Aufnahmen und eine zweite schlitzförmige Öffnung für Panoramaaufnahmen auf. Das Sensormodul kann dabei sowohl an der Halterung für Panoramaaufnahmen als auch an der Halterung für cephalometrische Aufnahmen befestigt werden.

Aus der DE 199 41 668 A1 ist eine Röntgeneinrichtung zur Erstellung von 3D-Aufnahmen bekannt. Auf die dort offenbarten Erläuterungen der Cone-Beam-Technik wird vollumfänglich zurückgegriffen.

Die Erstellung von Cone-Beam-Aufnahmen erfordert jedoch prinzipbedingt einen anderen Bildempfänger als den zur Erstellung der Schichtaufnahme, der üblicherweise als nach dem TDI-Prinzip betriebener CCD-Sensor ausgebildet ist. Dies gilt auch bei Bildempfängern, die Einzelbilder in Form eines Flächenbildes bereitstellen, welche nachträglich rechnerunterstützt zu einer entsprechenden Schichtaufnahme mit der geforderten Tiefenschärfe verrechnet werden. Derartige Bildempfänger sind beispielsweise CMOS-Detektoren.

Obwohl im Stand der Technik bereits vorgesehen ist, dass die für die Erstellung der Panorama-Schichtaufnahme verwendete Kamera durch Umstecken auch zur Erstellung einer Ceph-Aufnahme verwendet werden kann, ist für die Erstellung einer 3D-Aufnahme nach wie vor eine weitere Röntgeneinrichtung erforderlich.

### Darstellung der Erfindung

Gemäß der Erfindung wird eine Röntgeneinrichtung mit einer röntgenstrahlenempfindlichen Kamera zur Erstellung von dentalen Panoramaschichtaufnahmen vorgeschlagen.

Weiterhin ist in der Kamera ein erster Bildempfänger für die Erstellung einer Panoramaschichtaufnahme als Zeilensensor ausgebildet, der eine Länge aufweist, die ein Vielfaches größer als seine Breite ist. Desweiteren ist ein zweiter Bildempfänger zur Erstellung einer 2D-Aufnahme vorgesehen, wobei der zweite Bildempfänger ein Flächensensor ist. Es sind weiterhin Mittel zur Erstellung von 3D-Aufnahmen eines Teilvolumens des Kiefernbogens vorhanden, die dazu eingerichtet sind, unter Verwendung der Conebeam-Technik mit den dazugehörigen Rekonstruktionsalgorithmen mehrere 2D-Aufnahmen aus unterschiedlichen Richtungen aufzunehmen und daraus eine 3D-Aufnahme zu berechnen.

Weiterhin sind Verstellmittel vorhanden, mittels derer der erste Bildempfänger oder der zweite Bildempfänger wahlweise in den Strahlengang eines Röntgenstrahlers bringbar ist.

Mit einer derartigen Röntgeneinrichtung lassen sich zum Beispiel sowohl mit einem CCD-Sensor, der im TDI-Modus betrieben wird, Panoramaschichtaufnahmen als auch 3D-Aufnahmen eines Volumens unter Verwendung der Conebeam-Technik mit den dazugehörigen Rekonstruktionsalgorithmen erstellen.

Die US 2003/0030721 A1 gibt keinen Hinweis auf die Erstellung von 3D-Aufnahmen.

Vorteilhafterweise sind Steuerungsmittel vorhanden, so dass in der 3D-Aufnahme ein einen Ausschnitt aus der Panoramaschichtaufnahme umfassendes Teilvolumen erfassbar ist.

Vorteilhafterweise sind Verstellmittel und/oder Steuermittel vorhanden, mittels derer die Kamera und der Röntgenstrahler so verstellbar ist, dass der Drehmittelpunkt im aufzunehmenden Teilvolumen liegt. Vorteilhafterweise sind die Kamera und der Röntgenstrahler dazu an einem gemeinsamen Träger befestigt, wie dies an sich für Röntgeneinrichtungen zur Erstellung von Panoramaschichtaufnahmen bekannt ist.

Vorteilhafterweise sind mit der Kamera zusammenwirkende Verstellmittel vorgesehen, wobei die Verstellmittel im Gehäuse der Kamera oder in einer Kupplung zwischen der Kamera und dem Träger oder an dem Träger selbst vorgesehen sein können.

Bei im Gehäuse angeordneten Verstellmitteln sind diese gegen äußere Einflüssen geschützt. Bei an dem Träger vorgesehenen Verstellmitteln steht ein relativ großer Bauraum zur Verfügung und die Kamera kann kleiner und leichter ausgeführt sein.

Gemäß einer Weiterbildung kann die Röntgeneinrichtung zusätzlich mit einer Einrichtung für die Erstellung von Fernröntgenaufnahmen mit einem weiteren Bildempfänger versehen sein. Die Kamera ist bei Ausrichtung des Röntgenstrahlers zur Erstellung der Fernröntgenaufnahme im Bereich des Strahlengangs zwischen dem Röntgenstrahler und dem Bildempfänger der Einrichtung für die Erstellung von Fernröntgenaufnahmen angeordnet und ist in diesem Bereich strahlendurchlässig.

Alternativ dazu kann der Verstellweg so bemessen sein, dass bei Ausrichtung des Röntgenstrahlers zur Erstellung der Fernröntgenaufnahme die Kamera aus dem Strahlengang zwischen dem Röntgenstrahler und dem Bildempfänger der Einrichtung für die Erstellung von Fernröntgenaufnahmen herausführbar ist

Beides hat den Vorteil, dass auch bei einem Wechsel der Aufnahmeart von Nahröntgen (PAN/3D) zu Fernröntgen (Ceph) ein manueller Eingriff nicht erforderlich ist.

Vorteilhafterweise kann die Kamera exzentrisch verstellbar gehaltert sein und in einer ersten Stellung den Bildempfänger für die Erstellung einer Panoramaschichtaufnahme und in einer zweiten Stellung den Bildempfänger für die Erstellung einer 3D-Aufnahme positioniert werden. Der Röntgenstrahlfächer trifft dann den für die Erstellung der entsprechenden Aufnahme vorgesehenen Bildempfänger.

Eine röntgenstrahlenempfindliche Kamera umfasst einen ersten röntgenstrahlenempfindlichen Bildempfänger zur Erstellung einer Schichtaufnahme. Zur Erstellung von flächigen Aufnahmen ist ein zweiter röntgenstrahlenempfindlicher Bildempfänger vorgesehen.

Eine derartige Kamera ist daher außer zur Erstellung von Panorama-Schichtaufnahmen auch zur Erstellung von 3D-Aufnahmen geeignet. Damit lassen sich mit einer einzigen Kamera PAN- oder 3D-Aufnahmen durchführen.

Eine derartige Kamera ist somit zur Erstellung von unterschiedlichen Arten von Röntgenaufnahmen geeignet.

Dabei können die beiden Bildempfänger in einem gemeinsamen Gehäuse der Kamera angeordnet sein. Dies hat den Vorteil, dass eine Schnittstelle für die mechanische und elektrische Verbindung gemeinsame Befestigung ausreicht.

Der zweite Bildempfänger kann in einer ersten Abmessung seiner für die Bilderfassung vorgesehenen aktiven Fläche mindestens doppelt so groß wie der erste Bildempfänger sein. Darüber hinaus kann der zweite Bildempfänger in einer zweiten Abmessung höchstens halb so groß wie der erste Bildempfänger sein. Dies hat den Vorteil, dass bestehende längliche Zeilensensoren in den Abmessungen für PAN- oder Ceph-Aufnahmen einerseits und bestehende Flächensensoren in der für 3D-Aufnahmen erforderlichen Breite andererseits als Bildempfänger verwendet werden können. Es ist nicht erforderlich, einen PAN-Sensor in der für die Erstellung von 3D-Aufnahmen erforderlichen Breite vorzusehen, der wesentlich teurer wäre als die beiden einzelnen Sensoren zusammen.

Darüber hinaus kann bei der Kamera der zweite Bildempfänger seitlich neben dem ersten Bildempfänger angeordnet sein. Dadurch wird die Schulterfreiheit des Patienten, von dem eine Röntgenaufnahme zu erstellen ist, nicht durch die Kamera eingeschränkt.

Bei einer weiteren Kamera ist der zweite Bildempfänger auf der Rückseite des ersten Bildempfänger angeordnet. Eine derartige Kamera kann in herkömmliche Röntgengeräte zur Erstellung von PAN-Aufnahmen eingebaut werden und somit eine Nachrüstung zur Erstellung von 3D-Aufnahmen bereit stellen, insbesondere dann, wenn die Kamera in ihrer Ausrichtung zum Röntgenstrahler umgedreht werden kann.

Die US 2003/0030721 A1 gibt keinen Hinweis auf Verstellmittel, um seitlich benachbarte oder an ihrer Rückseite angeordnete Bildempfänger in den Strahlengang des Röntgenstrahlers zu bringen.

Die die Kamera kann so ausgebildet sein, dass der zweite Bildempfänger nachrüstbar ist. In diesem Fall ist es möglich, ein Röntgengerät zunächst mit der Kamera zur Erstellung von PAN-Aufnahmen auszustatten und erst bei Bedarf den zweiten Bildempfänger zur Erstellung von Multischicht-Aufnahmen in die Kamera einzusetzen.

Die Verstellmittel und die beiden Bildempfänger können in einem gemeinsamen Gehäuse der Kamera vorgesehen sein oder am Gehäuse der Kamera und im Bereich einer Kupplung zu einer Anbringung der Kamera an einem Träger, wobei dann die Kamera insgesamt gegenüber der Kupplung verstellbar ist. Im letzteren Fall ist es möglich, die Stellung der Kamera auch von außen ohne weiteres optisch zu kontrollieren und festzustellen, ob der richtige Sensor in die für die Aufnahme entsprechende Position gerückt ist. Weiterhin kann das Kameragehäuse kompakter gehalten werden, als wenn die Verstellung der Sensoren innerhalb des gesamten Kameragehäuses erfolgt.

Wenn die Kamera einen strahlendurchlässigen Bereich aufweist, ist es möglich, die Kamera im Röntgenstrahlfächer einer weiteren zu erstellenden Aufnahme zu belassen, ohne dass die Bilderstellung wesentlich beeinträchtigt wird. Dadurch kann die Kamera an ihrem Platz verbleiben und muss nicht entfernt werden.

Gemäß einer Ausführung ist der strahlendurchlässige Bereich zwischen dem ersten und dem zweiten Bildempfänger angeordnet.

Gemäß einer anderen Ausführung ist der strahlendurchlässige Bereich neben dem ersten und dem zweiten Bildempfänger angeordnet.

### Kurzbeschreibung der Zeichnung

In der Zeichnung ist ein Ausführungsbeispiel der Erfindung dargestellt. Es zeigt die
- Fig. 1a,b: eine Kamera mit zwei unterschiedlichen Bildempfängern, die nebeneinander angeordnet sind, die
- Fig. 2a,b: eine Kamera mit zwei unterschiedlichen Sensoren, die rückseitig angeordnet sind,
- Fig. 3a,b: einen ersten und zweiten Verstellmechanismus zum Verschieben der Sensoren innerhalb eines Kameragehäuses bzw, des Kameragehäuses, die
- Fig. 4a: ein Prinzipbild eines Röntgeneinrichtung zur Erstellung von PAN- und TSA-Aufnahmen gemäß der Erfindung in einer ersten Aufnahmesituation (PAN), die
- Fig. 4b: die Röntgeneinrichtung gemäß Fig. 5a in einer zweiten Aufnahmeposition (TSA), die

- Fig. 4c: eine weitere Röntgeneinrichtung mit einer dritten Aufnahmeposition (Ceph), die
- Fig. 4d: eine weitere Röntgeneinrichtung mit einer verstellbaren Primärblende für drei Aufnahmepositionen, die
- Fig. 4e,f: Schemazeichnungen von verschiedenen Aufnahmesituationen, die
- Fig. 5: eine weitere Schemazeichnung mit einer exzentrisch verschwenkbaren Kamera.

### Ausführungsbeispiel

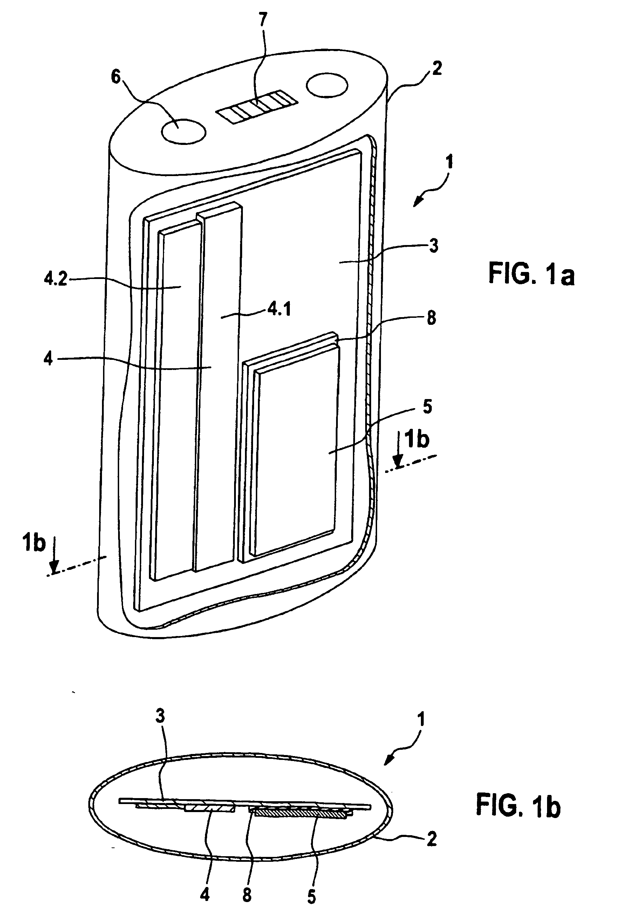

In Fig. 1a ist eine Kamera 1 in einer perspektivischen Ansicht dargestellt. Die Kamera 1 weist ein Gehäuse 2 auf, in welchem eine Platine 3 untergebracht ist. Auf der Platine 3 ist ein erster Bildempfänger 4 in Form eines Zeilensensors vorgesehen, der im Ausführungsbeispiel als CCD-Sensor ausgebildet ist und eine Länge aufweist, die ein Vielfaches größer ist als seine Breite. Der Bildempfänger 4 lässt sich in einen Bildempfangsbereich in Form eines CCD-Sensors 4.1 und eine Ausleseelektronik 4.2 unterteilen.

Derartige Ausführungen eines Bildempfängers sind im Stand der Technik hinlänglich bekannt. Grundsätzlich lassen sich auch Bildempfänger, die Einzelbilder in Form eines Flächenbildes bereitstellen, wie CMOS-Sensoren, verwenden.

Neben dem ersten Bildempfänger 4 ist ein weiterer Bildempfänger 5 vorgesehen, der als Flächensensor ausgebildet ist und aus dem mit hoher Geschwindigkeit Vollbilder ausgelesen werden können. Dieser Bildempfänger 4 ist ebenfalls auf dem Träger 3 angeordnet. Die räumlichen Abmessungen sind beispielsweise 60 mm x 60 mm (Höhe mal Breite) oder etwa 60 mm x 80 mm, so dass der Bildempfänger auch eher querliegend angeordnet sein kann. Es hat sich dabei gezeigt, dass ein Teilvolumen von 60 mm x 60 mm x 60 mm ausreichend ist, um die aufzunehmenden Bereiche hinreichend zu erfassen. Die genauen Abmessungen sind so auszuwählen, dass die Abmessungen des aufzunehmenden Teilvolumens erreicht werden.

Das Gehäuse 2 ist mit mechanischen und elektrischen Anschlussmitteln 6, 7 ausgestattet, sodass die Kamera 1 an einer nicht dargestellten, herkömmlichen Trägerstruktur befestigt werden kann.

In Fig. 1b ist ein Querschnitt durch die Kamera 1 gemäß der Schnittlinie aus Fig. 1a dargestellt. In dem Gehäuse 2 ist die Platine 3 mit dem ersten Bildempfänger 4 und dem zweiten Bildempfänger 5 dargestellt, wobei der zweite Bildempfänger 5 in eine Aufnahmevorrichtung 8 der Platine 3 eingesetzt ist.

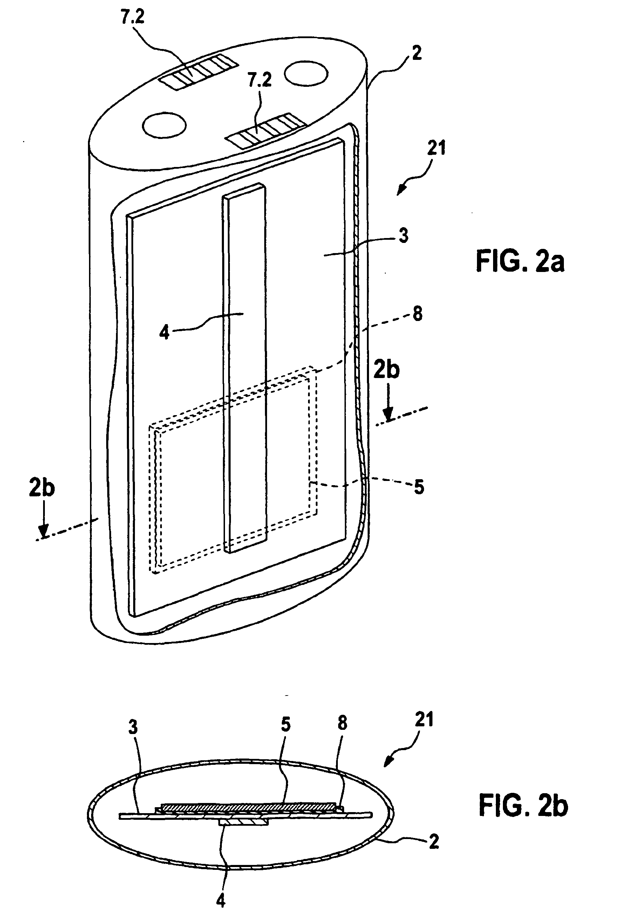

In Fig. 2a ist eine Kamera 21 dargestellt, welche wiederum ein Gehäuse 2 und eine Platine 3 aufweist, wobei auf der Platine 3 der erste Bildempfänger 4 angeordnet ist. Auf der Rückseite der Platine 3 ist der zweite Bildempfänger 5 angeordnet, dargestellt durch die gestrichelte Linie.

Um eine elektrische Kontaktierung auch im Falle des Umdrehens der Kamera 21 zu ermöglichen, ist der elektrische Kontakt 7 zweifach ausgeführt als 7.1 und 7.2. Diese doppelte Kontaktierung kann selbstverständlich auch im Bereich der nicht dargestellten Kamerakupplung an einer Röntgeneinrichtung vorgesehen sein.

Bei einer Drehung der Kamera etwa gemäß Fig. 6 ist eine umsteckbare Verbindung nicht erforderlich. Diese Drehung kann motorisch oder von Hand erfolgen.

In der Schnittzeichnung gemäß Fig. 2b ist die Anordnung der beiden Bildempfänger 4, 5 mit ihrer Rückseite zueinander, also auf beiden Seiten des Trägers 3 ohne weiteres zu erkennen. Der Bildempfänger 5 ist dabei in die Haltevorrichtung 8 eingebracht.

Da ein Röntgengerät zur Erstellung einer Panorama-Schichtaufnahme aufgrund der Häufigkeit der Erstellung derartiger Aufnahmen als Grundgerät angesehen wird, kann die Kamera so ausgeführt sein, dass der Bildempfänger 5 für die 3D-Aufnahme nachrüstbar ist. Eine Nachrüstung kann beispielsweise so geschehen, dass das Gehäuse geöffnet wird und der Bildempfänger 5 an eine entsprechende Stelle 8 gesteckt wird und gegebenenfalls weitere elektrische oder mechanische Verbindungen hergestellt werden.

Röntgengeräte zur Erstellung von Panorama-Schichtaufnahmen haben im Stand der Technik eine feste Kopplung zwischen dem Röntgenstrahler einerseits und dem Empfänger andererseits, sodass beide gemeinsam bewegt werden. In der Regel ist der Empfänger als solcher starr an dem gemeinsamen Träger zusammen mit dem Röntgenstrahler befestigt.

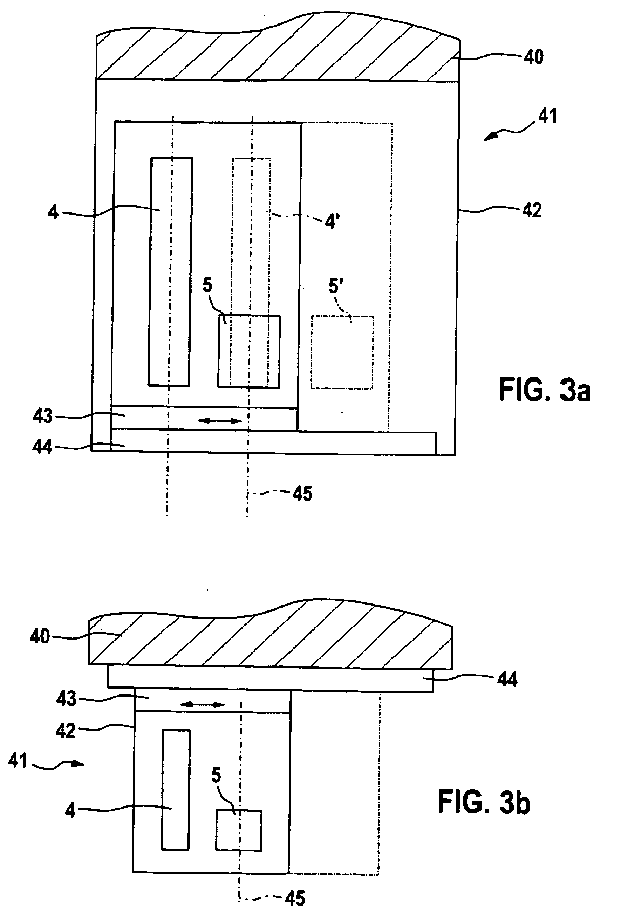

In den Fig. 3a, b ist ein erster und zweiter Verstellmechanismus zum Verschieben der Bildempfänger dargestellt.

Die an einer Trägerstruktur 40 befestigte Kamera 41 weist ein Gehäuse 42 auf, in welchem die Bildempfänger 4, 5 über einen Verstellmechanismus in Form eines Schlittens 43, der auf einer Verstellbahn 44 geführt wird, dargestellt. Die Bildempfänger 4, 5 lassen sich dabei über den Schlitten 43 und den Verstellmechanismus 44 von der dargestellten Position in die gestrichelte Position 4', 5' bringen, sodass an Stelle des Flächensensors des Bildempfängers 5 der Zeilendetektor des Bildempfängers 4 in den Strahlenfächer des Röntgenstrahls, dargestellt durch die Linie 45 gelangt.

In Fig. 3b ist der Verstellmechanismus zwischen einer Kamera 41 und dem Träger 40 angeordnet. Die Kamera 41 ist über ihr Gehäuse 42 mit der Trägerstruktur 40 verschiebbar verbunden, dargestellt durch den an der Kamera angeordneten Schlitten 43 und die an der Trägerstruktur 40 angeordnete Verstellbahn 44. Damit lässt sich die gesamte Kamera 41 von der dargestellten Position in die durch die gestrichelte Linie dargestellte Position verschieben, sodass der Strahlenfächer des Röntgenstrahls, wiederum dargestellt durch die Linie 45, vom Bildempfänger 5 auf den Bildempfänger 4 ausgerichtet ist.

Die Befestigung der Kamera 41 erfolgt über eine Kupplung, wobei bereits die Kupplungsmittel selbst Verstellmittel beinhalten können. Dies ist jedoch nicht dargestellt.

Mit einem motorisch verstellbaren Kamerahalter kann alternativ eine 3D-Aufnahme erstellt werden, wobei der Sensor je nach der vorgegebenen Betriebsart verstellt wird. Der motorisch verstellbare Kamerahalter stellt die Verbindung zwischen der Kupplung der Kamera und dem Träger her. Er kann so ausgeführt sein, dass die Kamera mit Kupplung auf einer Verschiebebahn oder mittels einer Schwenkvorrichtung bewegt werden kann. So kann die Kamera in die optimale Position am Gerät automatisiert verfahren werden. Dadurch ist eine direkte Aufnahmefolge einer PAN-Aufnahme mit anschließender Multischicht-Aufnahme ohne zusätzlichen Eingriff durch den Bediener möglich.

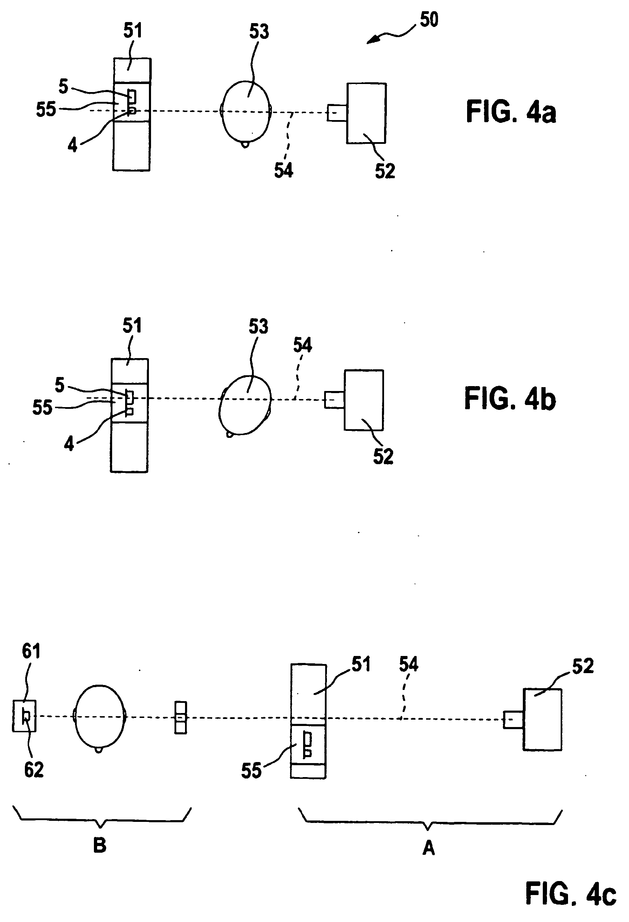

In Fig. 4a sind wesentliche Teile eines Röntgengerätes 50 dargestellt, nämlich eine Aufnahmeeinrichtung mit einer Aufnahmeeinheit 51 und ein Röntgenstrahler 52, wobei in dem Strahlengang zwischen Röntgenstrahler 52 und der Aufnahmeeinheit 51 das zu untersuchende Objekt in Form eines Patientenkopfes angeordnet ist. Zur Erstellung einer Panorama-Schichtaufnahme ist der aus dem Röntgenstrahler 52 austretende Röntgenstrahl 54 auf den als Zeilendetektor ausgebildeten Bildempfänger 4 ausgerichtet, sodass die erforderliche Länge zur Erstellung einer Panorama-Schichtaufnahme des oberen und unteren Kieferbogens bereit gestellt ist.

Der Bildempfänger 5 in Form des Flächensensors befindet sich hingegen in einer Ruhestellung außerhalb des Röntgenstrahls 54.

In Fig. 4b ist die Aufnahmesituation zur Erstellung einer 3D-Aufnahme eines interessierenden Teilbereichs des Kieferbogens, beispielsweise ein einzelner Zahn, dargestellt. Die an der Aufnahmeeinheit 51 angeordnete Kamera ist nun so ausgerichtet, dass der Bildempfänger 5 vom Röntgenstrahl 54 belichtet wird, wohingegen sich der Bildempfänger 4 in einer Ruhestellung befindet. Der eigentliche Aufnahmeablauf entspricht dem in der DE 199 41 668 A1 zu Fig. 1, 2 beschriebenen Ablauf.

Bei einer Kamera mit einer Sensoranordnung gemäß Fig. 2a, b befände sich dementsprechend einmal der Bildempfänger 4 dem Röntgenstrahler zugewandt angeordnet, dass andere mal der Bildempfänger 5. Dabei kann die Kamera durch motorische Verstellmittel automatisch umgedreht werden.

Für den Fachmann selbstverständlich, aber in den Figuren nicht immer dargestellt ist die Verwendung einer Primärblende mit mechanisch starr vorgegebene Öffnungen oder eine durch bewegliche, nicht dargestellte Strahlbegrenzungselemente einstellbare Öffnung zur Begrenzung des Röntgenstrahls, wobei die Ausdehnung des Röntgenstrahls im wesentlichen dem bildempfindlichen Bereich des Bildempfängers 4 bzw. 5 entspricht und bei Beachtung der einschlägigen Normen sogar vollständig auf der bildempfindlichen Fläche des Bildempfängers 4 bzw. 5 auftrifft. Dadurch werden unnötige Strahlenbelastungen mit nicht für die Bilderzeugung notwendigen Röntgenstrahlen vermieden.

In Fig. 4c ist ein Prinzipbild zur Erstellung einer Ceph-Aufnahme dargestellt.

Die Erstellung einer Ceph-Aufnahme kann in einer mit einer PAN-Einheit "A" und einer Ceph-Einheit "B" ausgerüsteten Röntgeneinrichtung so durchgeführt werden, dass für die Erstellung der Ceph-Aufnahme eine eigene Kamera 61 mit einem Bildempfänger 62 mit einem entsprechend langem Sensor in die Ceph-Position eingebracht ist. Die Kamera 55 zur Erstellung der PAN- und 3D-Aufnahme ist so verstellt, dass der aus dem Röntgenstrahler 52 austretende Röntgenstrahlfächer 54 am Gehäuse dieser Kamera 55 vorbei geht.

Wird auf einen eigenen Ceph-Sensor verzichtet, so kann ein manuelles Umstecken der ersten Kamera 55 erfolgen, wenn der darin befindliche Bildempfänger für die Erstellung der Pan-Aufnahme entsprechend lang ausgebildet ist, um auch den für die Erstellung der Ceph-Aufnahme relevanten Bereich abzudecken.

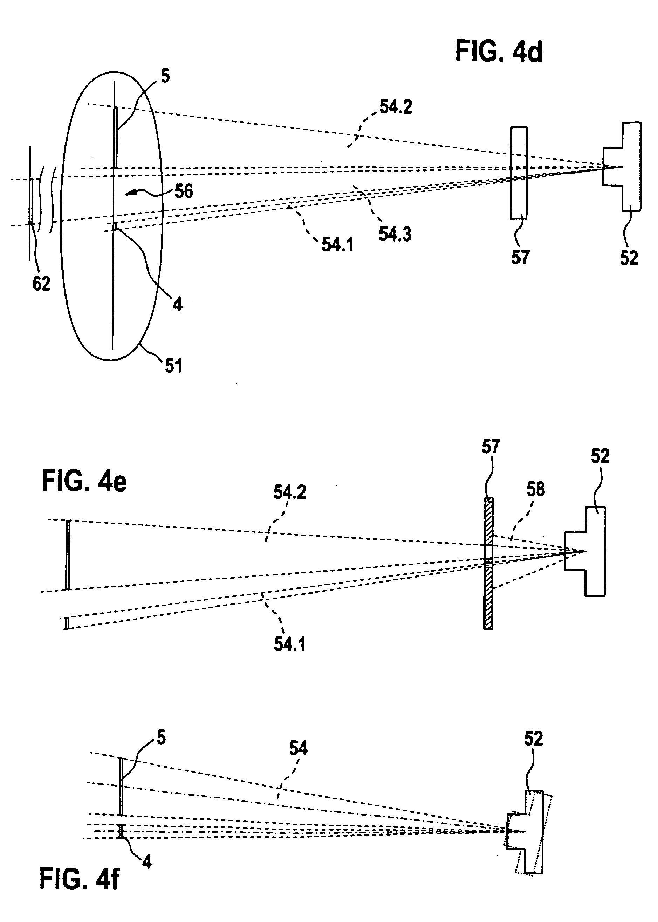

In Fig. 4d ist eine Aufnahmeeinheit 51 gezeigt, bei der zwischen den beiden Bildempfängern 4, 5 ein für Röntgenstrahlen durchlässiger Bereich 56 vorhanden ist. Die Abmessung des Bereichs 56 ist so bemessen, dass ein vom Röntgenstrahler 52 ausgehender Röntgenstrahlfächer 54 im wesentlichen unbeeinflusst durch die Kamera hindurchdringt.

In diesem Ausführungsbeispiel ist die Kamera feststehend und der Röntgenstrahlfächer 54.1 - 54.3 wird über eine verstellbare Primärblende 57 auf den jeweiligen Bildempfänger 4, 5, 62 ausgerichtet. Die Primärblende 57 ist dabei in ihren geometrischen Abmessungen auf die jeweils zu erstellende Aufnahme abgestimmt. Für die Erstellung einer PAN-Aufnahme ist die Breite beispielsweise 0,9 mm.

In Fig. 4e ist dieses Prinzip im Detail dargestellt. Die Primärblende 57 weist hier zwei Öffnungen auf, die den den verschiedenen Aufnahmearten entsprechenden Röntgenstrahlfächer 54.1, 54.2 freigeben können. Der jeweils andere Röntgenstrahlfächer ist zur Erstellung der Aufnahme selbstverständlich ausgeblendet. Der vom Röntgenstrahler bereitgestellte Strahlenkegel 58 ist hinreichend groß, um die gewünschten Röntgenstrahlfächer 54.1, 54.2 oder gegebenenfalls den Röntgenstrahlfächer für eine Fernröntgenaufnahme bereitzustellen.

Anstelle der Strahlaufteilung des aus dem Röntgenstrahler 52 austretenden Röntgenstrahlfächers 58 mittels einer verstellbaren Primärblende kann auch der Röntgenstrahler 52 über Verstellmittel auf den jeweils gewünschten Bildempfänger 4, 5 ausgerichtet werden, dargestellt in Fig. 4f. Eine derartige Verstellung ist für PAN/Ceph-Kombigeräte bereits bekannt. Die Verstellung kann durch Verschieben oder wie dargestellt durch Verschwenken erfolgen. Der Vorteil hierbei ist, dass stets der Zentralstrahl des Röntgenstrahlfächers 58 im Röntgenstrahlfächer 54 liegt.

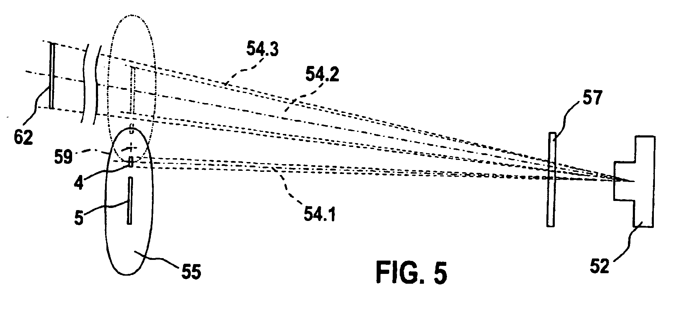

Bei der in Fig. 5 dargestellten exzentrischen Anordnung der Kamera 2 kann in einer ersten Ausrichtung der Kamera 2 eine PAN-Aufnahme erstellt werden, wobei der Bildempfänger 4 in dem Röntgenstrahlfächer 54.1 liegt. In dieser Ausrichtung der Kamera 2 kann auch eine Ceph-Aufnahme erstellt werden, da der Röntgenstrahlfächer 54.3 an der Kamera 2 vorbeistrahlt. In der gestrichelt dargestellten Ausrichtung der Kamera 2, die durch Drehen um den Exzenterpunkt 59 erreicht wird, kann eine 3D-Aufnahme erstellt werden. Dabei ist der Bildempfänger 4 näher am Röntgenstrahlfächer 54.3 der Ceph-Aufnahme angeordnet als der Bildempfänger 5.

Die gezeigte Anordnung hat den Vorteil, dass für die Erstellung der Ceph-Aufnahme ein kurzer Ausleger für die Ceph-Kamera ausreicht, da der Röntgenstrahlfächer 54.3 wandnah bleibt.

Grundsätzlich gilt, dass die Primärblende zur Erstellung einer PAN-Aufnahme, einer 3D-Aufnahme und einer Ceph-Aufnahme jeweils verschieden ist und dass eine Aufnahme nur mit einer einzige Aufnahmeart erstellt wird. Sind in den Ausführungsbeispielen mehrere Röntgenstrahlenfächer gleichzeitig dargestellt, so dient dies lediglich zur Verdeutlichung der geometrischen Verhältnisse. Die Primärblende ist aber so ausgebildet und wird so eingestellt, dass der gewünschte Bildempfänger mit dem für die Bilderstellung vorgesehenen Röntgenstrahlfächer beaufschlagt wird.

## Patentansprüche

1. Röntgeneinrichtung mit einer röntgenstrahlenempfindlichen Kamera (1) zur Erstellung von dentalen Panoramaschichtaufnahmen, wobei die Kamera (1) über einen ersten und einen zweiten Bildempfänger (4, 5) verfügt, wobei der erste Bildempfänger (4) für die Erstellung einer Panoramaschichtaufnahme als Zeilensensor ausgebildet ist, der eine Länge aufweist, die ein Vielfaches größer als seine Breite ist und der zweite Bildempfänger (5) zur Erstellung einer 2D-Aufnahme vorgesehen ist, wobei der zweite Bildempfänger (5) ein Flächensensor ist und wobei Mittel zur Erstellung von 3D-Aufnahmen eines Teilvolumens des Kieferbogens vorhanden sind, die dazu eingerichtet sind, unter Verwendung der Conebeam-Technik mit den dazugehörigen Rekonstruktionsalgorithmen mehrere 2D-Aufnahmen aus unterschiedlichen Richtungen aufzunehmen und daraus eine 3D-Aufnahme zu berechnen, wobei weiterhin Verstellmittel (44) vorhanden sind, mittels derer der erste Bildempfänger (4) oder der zweite Bildempfänger (5) wahlweise in den Strahlengang (54) eines Röntgenstrahlers (52) bringbar ist.

2. Röntgeneinrichtung nach Anspruch 1, **dadurch gekennzeichnet, dass** Steuerungsmittel vorhanden sind, so dass in der 3D-Aufnahme ein einen Ausschnitt aus der Panoramaschichtaufnahme umfassendes Teilvolumen erfassbar ist.

3. Röntgeneinrichtung nach einem der Ansprüche 1 bis 2,
**dadurch gekennzeichnet, dass** Verstellmittel (44) und/oder Steuermittel vorhanden sind, mittels derer die Kamera (1) und der Röntgenstrahler (52) so verstellbar ist, dass der Drehmittelpunkt im aufzunehmenden Teilvolumen liegt.

4. Röntgeneinrichtung nach einem der Ansprüche 1 bis 3,
**dadurch gekennzeichnet, dass** die Verstellmittel (44) im Gehäuse (2) der Kamera (1) oder in einem Kupplungsstück zwischen der Kamera (1) und einem Träger (40) oder an dem Träger (40) selbst vorgesehen sind.

5. Röntgeneinrichtung nach einem der Ansprüche 1 bis 4,
**dadurch gekennzeichnet, dass** zusätzlich eine Einrichtung (61) für die Erstellung von Fernröntgenaufnahmen mit einem weiteren Bildempfänger (62) vorgesehen ist und dass die Kamera (1) bei Ausrichtung des Röntgenstrahlers (52) zur Erstellung der Fernröntgenaufnahme im Bereich des Strahlengangs zwischen dem Röntgenstrahler (52) und dem Bildempfänger (62) der Einrichtung für die Erstellung von Fernröntgenaufnahmen (61) angeordnet ist und in diesem Bereich (56) strahlendurchlässig ist.

6. Röntgeneinrichtung nach einem der Ansprüche 1 bis 4,
**dadurch gekennzeichnet, dass** zusätzlich eine Einrichtung (61) für die Erstellung von Fernröntgenaufnahmen mit einem weiteren Bildempfänger (62) vorgesehen ist und dass der Verstellweg so bemessen ist, dass bei Ausrichtung des Röntgenstrahlers (52) zur Erstellung der Fernröntgenaufnahme die Kamera (1) aus dem Strahlengang zwischen dem Röntgenstrahler (52) und dem Bildempfänger (62) der Einrichtung (61) für die Erstellung von Fernröntgenaufnahmen herausführbar ausgebildet ist.

7. Röntgeneinrichtung nach einem der Ansprüche 1 bis 6,
**dadurch gekennzeichnet, dass** die Kamera (1) exzentrisch verstellbar gehaltert ist und dass in einer ersten Stellung der Bildempfänger (4) für die Erstellung einer Panoramaschichtaufnahme und in einer zweiten Stellung der Bildempfänger (5) für die Erstellung einer 3D-Aufnahme im Röntgenstrahlfächer (54) positioniert ist.

## Claims

1. An X-ray system having an X-ray-sensitive camera (1) for the creation of dental panoramic tomographic images, which camera (1) includes a first and a second image detector (4, 5), **wherein** said first image detector (4) is designed as a line sensor with a length that is a multiple greater than its width for the creation of a panoramic tomographic image, and said second image detector (5) is provided for the creation of a 2D image, wherein said second image detector (5) is a surface sensor, and means are provided for the creation of 3D images of a subvolume of the mandibular arch, which means are set up to create several 2D images from different directions and compute a 3D image therefrom using the cone beam technique with associated reconstruction algorithms, and adjustment means (44) are also provided by means of which said first image detector (4) or said second image detector (5) can be moved as desired into the optical path (54) of an X-ray emitter (52).

2. An X-ray system as defined in claim 1, wherein control means are provided such that within the 3D image a subvolume comprising a portion of the panoramic tomographic image can be imaged.

3. An X-ray system as defined in any one of claims 1 to 2, wherein there are provided adjustment means (44) and/or control means by means of which said camera (1) and said X-ray emitter (52) can be adjusted such that the center of rotation lies in the subvolume to be imaged.

4. An X-ray system as defined in any one of claims 1 to 3, wherein said adjustment means (44) are provided in said casing (2) of said camera (1) or in connecting means between said camera (1) and a support (40) or on said support (40) itself.

5. An X-ray system as defined in any one of claims 1 to 4, wherein there is additionally provided an installation (61) for the creation of teleradiographic images with another image detector (62) and, when said X-ray emitter (52) is aligned for the creation of a teleradiographic image, said camera (1) is disposed in the region of the optical path between said X-ray emitter (52) and said image detector (62) of said installation (61) for the creation of teleradiographic images and is radiolucent in said region (56).

6. An X-ray system as defined in any one of claims 1 to 4, wherein there is additionally provided an installation (61) for the creation of teleradiographic images with another image detector (62) and the path of adjustment is such that, when the X-ray emitter (52) is aligned for the creation of a teleradiographic image, said camera (1) can be moved out of the optical path between said X-ray emitter (52) and said image detector (62) of said installation (61) for the creation of teleradiographic images.

7. An X-ray system as defined in any one of claims 1 to 6, wherein said camera (1) is mounted for eccentric displacement and, in a first position, said image detector (4) is positioned in the X-ray fan beam (54) for the creation of a panoramic tomographic image and, in a second position, said image detector (5) is positioned in the X-ray fan beam (54) for the creation of a 3D image.

## Revendications

1. Equipement radiologique doté d'une caméra (1) sensible aux rayons X pour l'élaboration d'orthopantomogrammes, la caméra (1) disposant d'un premier récepteur d'image et d'un second récepteur d'image (4, 5), le premier récepteur d'image (4) étant conçu, pour l'élaboration d'un orthopantomogramme, comme un détecteur de lignes, qui présente une longueur qui est plusieurs fois supérieure à sa largeur et le second récepteur d'image (5) étant prévu pour la réalisation d'un cliché en deux dimensions, le second récepteur d'image (5) étant un capteur de surface et des moyens étant présents pour la réalisation de clichés en trois dimensions d'un volume partiel d'un arc de mâchoire, lesquels sont agencés, en utilisant la technique de Conebeam avec les algorithmes de reconstitution correspondants, pour prendre plusieurs clichés en deux dimensions à partir de différentes directions et pour calculer un cliché en trois dimensions à partir de ceux-ci, des moyens de réglage (44) étant en outre présents, à l'aide desquels le premier récepteur d'image (4) ou le second récepteur d'image (5) peut être amené au choix dans le trajet du faisceau (54) d'un émetteur de rayons X (52).

2. Equipement radiologique selon la revendication 1, **caractérisé en ce que** des moyens de commande sont présents, de sorte qu'un volume partiel comprenant un extrait de l'orthopantomogramme peut être pris en compte dans le cliché en trois dimensions.

3. Equipement radiologique selon l'une des revendications 1 à 2, **caractérisé en ce que** des moyens de réglage (44) et/ou des moyens de commande sont présents, à l'aide desquels la caméra (1) et l'émetteur de rayons X (52) peuvent être réglés de telle sorte que le centre de rotation se situe dans le volume partiel à enregistrer.

4. Equipement radiologique selon l'une des revendications 1 à 3, **caractérisé en ce que** les moyens de réglage (44) sont prévus dans le boîtier (2) de la caméra (1) ou dans une pièce d'accouplement entre la caméra (1) et un support (40) ou sur le support (40) même.

5. Equipement radiologique selon l'une des revendications 1 à 4, **caractérisé en ce que**, en plus, un équipement (61) est prévu pour la réalisation de radiographies à distance avec un autre récepteur d'image (62) et **en ce que**, lorsque l'émetteur de rayons X (52) est orienté pour réaliser la radiographie à distance, la caméra (1) est disposée dans la zone du trajet du rayonnement entre l'émetteur de rayons X (52) et le récepteur d'image (62) de l'équipement pour la réalisation de radiographies à distance (61) et est transparente au rayonnement dans cette zone (56).

6. Equipement radiologique selon l'une des revendications 1 à 4, **caractérisé en ce que**, en plus, un équipement (61) est prévu pour la réalisation de radiographies à distance avec un autre récepteur d'image (62) et **en ce que** la course d'ajustage est dimensionnée de telle sorte que, lorsque l'émetteur de rayons X (52) est orienté pour réaliser la radiographie à distance, la caméra (1) est conçue de façon à pouvoir être guidée hors du trajet du rayonnement entre l'émetteur de rayons X (52) et le récepteur d'image (62) de l'équipement (61) pour la réalisation de radiographies à distance.

7. Equipement radiologique selon l'une quelconque des revendications 1 à 6, **caractérisé en ce que** la caméra (1) est maintenue de manière à pouvoir être réglée excentriquement et **en ce que**, dans une première position, le récepteur d'image (4) est positionné pour la réalisation d'un orthopantomogramme et, dans une deuxième position, le récepteur d'image (5) est positionné pour la réalisation d'un cliché en trois dimensions dans les compartiments de rayons X (54).
